Europäisches Patentamt

(19) European Patent Office

Office européen des brevets

(11) Publication number : **0 494 916 B1**

(12) **EUROPEAN PATENT SPECIFICATION**

(45) Date of publication of patent specification :
02.03.94 Bulletin 94/09

(21) Application number : 90914684.7

(22) Date of filing : 05.10.90

(86) International application number :
PCT/DK90/00256

(87) International publication number :
WO 91/04673 18.04.91 Gazette 91/09

(51) Int. Cl.$^5$ : **A23K 1/18, A23K 1/165**

(54) **FEED ADDITIVE FOR POULTRY AND POULTRY FEED.**

(30) Priority : 06.10.89 DK 4940/89

(43) Date of publication of application :
22.07.92 Bulletin 92/30

(45) Publication of the grant of the patent :
02.03.94 Bulletin 94/09

(84) Designated Contracting States :
AT BE CH DE DK ES FR GB IT LI LU NL SE

(56) References cited :
EP-A- 0 280 226
US-A- 3 462 275
Med. Fac. Landbouww. Rijksuniv. Gent, Vol.
53, No. 4a, 1988 W.D. Cowan: "Selection and
application of enzymes to improve utilisation
of animal feeds"
Derwent's abstract, No. 78-649 21A/36, SU 577
230, publ. week 7836, MOSC FOOD IND
TECHN; BIOTECH RES INST
Derwent's Abstract No. 36 259 K/15, SU 933
063, publ. week 8315, POULTRY RES INST
Chemical Abstracts, vol. 99, no. 23, 5 Decem-
ber 1983 (Colombus, Ohio, US), Isshiki, Y. et
al.:"Effect of pectinase, xylanase and cellul-
ase supplements on the utilisation of feed in
chicks", see pp. 652-653, abstract 193693d, &
Nippon Kakin Gakkaishi 1983, 20(4), 237-243
Chemical Abstracts, vol. 96, no. 13, 29 March
1982 (Colombus, Ohio, US), Hayashida, S. et
al.: "Production of thermostable cellulases
and raw starch-digesting amylases by fungi",
p. 387, abstract 100626j, & Adv. Biotechnol.
(Proc. Int. Ferment. Symp.) 1981, 3, 271-276

(56) References cited :
Chemical Abstracts, vol. 98, no. 15, 11 April
1983 (Colombus, Ohio, USA), Sandhu
D.K.:"Production of cellulase, xylanase and
pectinase by Trichoderma longibrachiatum on
different substrates", p. 483, abstract 124113,
& Trans. Br. Mycol. Soc. 1982, 79(3), 409-413
Chemical Abstracts, vol. 76, no. 7, 14 February
1972 (Colomvbus, Ohio, US), Bakai, S.M.: "Use
of fungal cellulase in the preparation of
feeds", p. 228, abstract 33005c, & Fermenty
Nar. Khoz. Med. 1971, 155-158

(73) Proprietor : NOVO NORDISK A/S
Novo Allé
DK-2880 Bagsvaerd (DK)
Proprietor : GUYOMARC'H NUTRITION
ANIMALE
BP 234
F-56006 Vannes Cédex (FR)

(72) Inventor : NGUYEN, Tan, Hung
Le Porlair
F-56890 Saint-Avé (FR)
Inventor : COWAN, David, William
Bolbrovej 82B
DK-2960 Rungsted Kyst (DK)
Inventor : JOERGENSEN, Ole, Bill
Vagtelvej 71
DK-2000 Koebenhavn F (DK)

(74) Representative : Bach, Niels et al
c/o Novo Nordisk A/S Patent Dept. Novo Allé
DK-2880 Bagsvaerd (DK)

## Description

The invention comprises a feed additive for poultry and a poultry feed. The feed additive is used as a medicament for alleviation of the malabsorption syndrome in broiler chickens.

Malabsorption syndrome is a problem observed in broilers in many countries of the world. This syndrome causes very important losses to the poultry industry. The affected flocks show several variable clinical symptoms:

- Lower digestion of feeds causing diarrhoea, particularly fats and fat soluble substances, or only wet litter,
- reduced fat digestability
- poor growth (runting or stunting syndrome)
- high feed:gain ratio
- brittle bones
- defective feathering
- defective pigmentation of the skin (pale bird syndrome): The yellow colour of affected birds is less than normal and the pigmentation is quite variable between birds of a same flock; this defective pigmentation is due to a bad utilization of the pigments called xanthophylls contained in feeds.

Sometimes only one of these symptoms exists in a flock. Sometimes several symptoms can be seen in the same flock.

The etiology of the syndrome is not well determined.

Several viruses have been cited as possible causal agents of the syndrome, e.g. Rotavirus, reovirus, coronavirus, adenovirus, calicivirus, parvovirus, and togavirus.

Mycotoxins have also been cited as possible causal agents of the syndrome.

The invention describes a feed additive for alleviation of the malabsorption syndrome in poultry and a poultry feed.

The feed additive for alleviation of the malabsorption syndrome in poultry according to the invention is characterized by the fact that it comprises a cellulase and a xylanase producible by means of *Humicola insolens* (DSM 1800) and/or *Trichoderma longibrachiatum* (NRRL 11460). NRRL 11460 was originally deposited as *Trichoderma reesei* and later reclassified as *Trichoderma longibrachiatum.* Surprisingly it has been found that this feed additive containing as the active component a specific cellulase/xylanase combination exhibits the ability effectively to alleviate the malabsorption syndrome in poultry.

In Chemical Abstract No. 98:124113g the production of a cellulase and a xylanase by means of *Trichoderma longibrachiatum* is described. However, no use of these enzymes are indicated.

In Chemical Abstract No. 99:193693d the use of cellulases and xylanases as feed components in chicken is described. However, cellulases and xylanases produced by means of *Humicola insolens* or *Trichoderma longibrachiatum* are not indicated.

Due to the fact that part of the malabsorption syndrome comprises a reduced fat digestibility, the obvious constituent of a feed additive for alleviation of the malabsorption syndrome would be a lipase. According to the invention, however, it surprisingly has been found that lipase does not alleviate the malabsorption syndrome to any significant degree. Even more surprisingly, as above indicated, it has been found, that the above indicated feed additive with the most specific cellulase/xylanase combination effectively alleviates the malabsorption syndrome, despite the fact that no corresponding theoretical explanation at present can be provided.

It belongs to the prior art that the growth of poultry can be improved by addition of cellulase and/or xylanase to the feed, vide e.g. SU patent no. 933.063 (referenced in Derwents abstract in World Patent Index Latest), and DK patent application no. 805/88, and selected cellulase/xylanase enzyme complexes for that purpose are described in SU patent no. 577.230 (referenced in Derwents abstract in World Patent Index), and DE 1.904.239. However, the feed:gain ratio of the prior art feeds are not satisfactory, and it has been found that the feed:gain ratio of the poultry feed according to the invention is significantly better than the feed:gain ratio of the prior art poultry feeds, vide Example 6. More important however, according to the invention we are able to offer an alleviation of the symptoms of the malabsorption syndrome which is not possible according to the prior art. There is also the advantage that due to this improvement less manure has to be disposed of according to the invention, whereby less pollution is generated according to the invention.

The poultry feed according to the invention is characterized by the fact that the poultry feed is composed of a normal poultry feed, to which a feed additive according to the invention has been added.

It is known from the prior art that microbial enzymes may improve the growth of chickens when such enzymes are incorporated into feeds and the range of enzymes includes amylase, cellulase, hemicellulase, glucanase, lipase, gumase, chitinase and cytolytic enzymes. However, the specific combination of the enzymes producible by means of *Humicola insolens* (DSM 1800) or *Trichoderma longibrachiatum* (NRRL 11460) is needed.

The present invention demonstrates how the addition of a selected cellulase/xylanase enzyme complex is able to alleviate the effects of malabsorption syndrome and return the performance of the chickens to their normal state. It is also demonstrated that traditional cellulases do not have the same effect.

The examples given below demonstrate the properties and advantages of the invention.

SP 343 is the *Humicola insolens* cellulase/xylanase complex from DSM 1800, and SP 431 is the xylanase from *Trichoderma longibrachiatum* (NRRL 11460).

## EXAMPLE 1

160 chicken were taken randomizedly, at 8 days of age, from a flock raised in an area where malabsorption syndrome was endemic.

These 160 chicken were afterwards raised in individual cages, on wired floor. Each cage was equipped with a feeder and a waterer in order to provide feed and water ad libitum.

The 160 chicken were divided in 4 lots of 40 birds. Each lot received one of these 4 experimental feeds:
1) Basal feed - wheat based (control)
2) Basal feed supplemented with enzyme SP 343 at 200 g/ton
3) Basal feed supplemented with enzyme SP 343 at 1000 g/ton
4) Basal feed supplemented with Lipozyme 10,000 L at 50 g/ton

After 8 days of experiment, feces were collected for each bird for 3 days. The collected feces of 2 birds consuming the same feed were pooled together: Results of the nutritional balance trial were obtained with 20 individual data for each feed.

Dry matter and fat were analysed in feeds and feces in order to determine dry matter and fat digestibility according to the well known procedure commonly used in animal nutrition.

The results were submitted to variance analysis and Duncan's comparison of the means.

The results obtained (table 1) show that the SP 343 enzyme improved dry matter and fat digestibility but that the lipase (Lipozyme 10000 L) was not able to improve digestibility of these components.

## Table 1

| Lots | Feed | Dry matter Digestibility (%) | Fat Digestibility (%) |
|---|---|---|---|
| 1 | Basal | 63.0±4.2 A | 43.1±17.1 A |
| 2 | Basal +Enzyme 200 g/t | 67.8±3.9 B | 54.8±14.7 B |
| 3 | Basal +Enzyme 1000 g/t | 68.7±3.4 B | 58.0±11.5 B |
| 4 | Basal +Lipase | 59.7±5.3 A | 36.6±17.4 A |
| Variance analysis | | $p \leq 0.001$ | $p \leq 0.001$ |

(In a same column, A and B means a significant difference ($p \leq 0.001$) with Duncan's test).

## EXAMPLE 2

320 chicken were taken, at 8 days of age, from a flock raised in an area where malabsorption syndrome was endemic.

The birds were divided in 4 lots of 80 birds.

The birds were raised in individual cages as previously described in Example 1.

Each lot of birds received one of these 4 experimental feeds, for three weeks (from 8 to 29 days of age):

1) Basal feed

2) Basal feed supplemented with enzyme SP 343 at 200 g/ton

3) Basal feed supplemented with enzyme SP 343 at 1000 g/ton

4) Basal feed supplemented with Lipozyme 10000 L at 50 g/ton

Body weight gain, feed intake and feed:gain ratio were measured for each bird from 8 to 29 days of age.

Results were submitted to statistical analysis as previously described.

The results (table 2) show that birds consuming the basal feed had high feed:gain ratio. The SP 343 enzyme improved growth, and decreased feed intake and feed:gain ratio significantly. The lipase enzyme added at a dose rate in excess of the lipase side activity present in SP 343 did not significantly change the feed:gain ratio.

## Table 2

| Lots | Feeds | Initial weight (g) | Final weight (g) | Weight gain (g) | Feed intake (g) | Feed:gain ratio |
|---|---|---|---|---|---|---|
| 1 | Basal | 142.4 | 925.8a (100.0) | 783.4a (100.0) | 1487.0a (100.0) | 1.904A (100.0) |
| 2 | Basal +Enzyme 200 g/t | 142.9 | 984.8ab (102.6) | 806.9ab (103.0) | 1479.8ab (99.5) | 1.842B (96.7) |
| 3 | Basal +Enzyme 1000 g/t | 143.0 | 960.7b (103.8) | 817.7b (104.4) | 1439.3b (96.8) | 1.767C (92.8) |
| 4 | Basal +Lipase 50 g/ton | 142.4 | 940.6ab (101.6) | 798.2ab (101.9) | 1504b (101.2) | 1.892A (99.4) |
| Variance analysis | | $p \leq 0.05$ | $p \leq 0.05$ | $p \leq 0.05$ | $p \leq 0.05$ | $p \leq 0.001$ |

In a same column, Duncan's test:
a, b :　　　$p \leq 0.05$
A, B, C :　　　$p \leq 0.001$

**EXAMPLE 3**

320 broilers were taken, at 20 days of age, from a farm located in an area where malabsorption syndrome was endemic.

After being weighed, they were distributed in 4 lots of 4 replications of 20 birds for each replication.

The birds were raised on floor pen with wood shaving litter (2 $m_2$/pen).

Each lot of birds received one of these 4 experimental feeds, from 20 to 41 days of age (21 days of experiment):

1) Basal feed 1 - maize base
2) Basal feed 1 supplemented with enzyme SP 343 at 1000 g/ton
3) Basal feed 2 - wheat base

5

4) Basal feed 2 supplemented with enzyme SP 343 at 1000 g/ton
The xanthophyll composition of the 2 basal feeds are on table 3.

<u>Table 3</u>

|                      | Maize base | Wheat base |
|----------------------|------------|------------|
| Lutein (ppm)         | 41.80      | 42.90      |
| Zeaxanthin (ppm)     | 13.00      | 12.00      |
| Canthaxanthin (ppm)  | 0.92       | 1.03       |

On the 14th day of the experiment, blood plasma was collected from 12 birds of each treatment (3 birds of each replication) for measuring the optical density by spectrophotometry (with a spectrophotometer Beckman DU 64) at 450 nm. The optical density of plasma reflects the absorbed xanthophyll content of the blood.

Weight gain, feed intake and feed:gain ratio of the birds were also measured for the 21 days of experiment.

After the 21 days of experiment all the birds were slaughtered in an industrial abattoir. After cooling for 24 hours, the pigmentation of the carcasses was ranked by 2 well trained quality controllers of the abattoir. Each bird was given a score:
1: badly pigmented
2: moderately pigmented
3: well pigmented
The average score was calculated for each treatment.

# RESULTS

## Optical Density of Plasma

### Table 4

| Lots | Feeds | Optical Density | Coefficient of Variation (%) |
|------|-------|-----------------|------------------------------|
| 1 | Maize base | 0.441 ± 0.173 | 39.3 |
| 2 | Maize base + enzyme | 0.605 ± 0.163 | 27.3 |
| 3 | Wheat base | 0.407 ± 0.173 | 43.7 |
| 4 | Wheat base + enzyme | 0.615 ± 0.117 | 19.0 |
| Variance analysis | | $p \leq 0.001$ | - |

Table 4 shows that optical density was significantly increased by the enzyme. There were therefore more xanthophylls in the blood.

Coefficient of variation of optical density was also lower with birds receiving enzyme: The xanthophyll contents of the blood were more homogenous with enzyme supplemented feeds than with control feeds.

Pigmentation of Carcasses

Table 5 shows that the pigmentation score of carcasses was increased by the enzyme. In basal feeds, 2 to 5 percents of birds were scored as badly pigmented, while there was no such scored bird with enzyme.

## Table 5

| Lots | Feeds | Score | Contr.1 %birds | Score | Contr.2 %birds | Score | Average Score |
|------|-------|-------|----------------|-------|----------------|-------|---------------|
| 1 | Maize base | 3<br>2<br>1 | 20<br>77<br>3 | 2.17 | 20<br>78<br>2 | 2.18 | 2.175 |
| 2 | Maize base<br>+<br>Enzyme | 3<br>2<br>1 | 31<br>69<br>0 | 2.31 | 25<br>75<br>0 | 2.25 | 2.280 |
| 3 | Wheat base | 3<br>2<br>1 | 14<br>84<br>2 | 2.12 | 15<br>80<br>5 | 2.10 | 2.110 |
| 4 | Wheat base<br>+<br>Enzyme | 3<br>2<br>1 | 29<br>71<br>0 | 2.29 | 30<br>70<br>0 | 2.30 | 2.295 |

Zootechnical Performances

## Table 6

| Lots | 1 | 2 | 3 | 4 |
|---|---|---|---|---|
| Feeds | Maize base | Maize base + enzyme | Wheat base | Wheat base + enzyme |
| Live weight (g) | | | | |
| initial | 510±5 | 512±8 | 520±1 | 1517±15 |
| final | 1651±4 | 1661±28 | 1656±39 | 1632±71 |
| Weight gain(g) | 1141±8 | 1150±25 | 1136±32 | 1114±57 |
| Feed intake(g) | 2570±79 | 2551±10 | 2608±123 | 2438±135 |
| Feed:gain ratio | 2.253±0.078 | 2.219±0.052 | 2.296±0.072 | 2.188±0.039 |

Table 6 shows that the enzyme:
- Improved slightly growth of birds with maize based feed.
- Decreased feed intake and feed:gain ratio for both kinds of feeds.

**EXAMPLE 4**

Digestibility Trials

Cecectomized cockerels are divided at random into groups of 6 birds. A basal wheat based feed is used supplemented with enzyme at the rate of 1 kg per ton. The birds are starved for 30 hours and then each fed 80 g of feed. Faeces are collected from each bird and then pooled for each treatment group. The pooled samples are oven dried at 70°C and then analysed by the current EEC methods except for fat which is analysed by acid hydrolysis.

The results in table 7a and 7b show that the specific cellulase/xylanase combination found in SP 343 and SP 431 are able to improve fat digestibility in the cecectomized cockerel but that lipase or standard cellulase from Tricoderma sp is not able to improve digestibility.

## Table 7a

| Enzyme | True Digestibility, % | |
|---|---|---|
| | Dry matter | Fat |
| Control | 72.95 | 87.78 |
| SP 343 | 72.87 | 90.98 |
| SP 431 | 74.04 | 91.59 |
| Lipolase | 71.62 | 87.98 |

## Table 7b

| Enzyme | True Digestibility, % | |
|---|---|---|
| | Dry matter | Fat |
| Control | 72.28 | 87.16 |
| SP 343 | 72.26 | 88.52 |
| Celluclast | 72.36 | 86.73 |

**EXAMPLE 5**

Comparison of the Effect of a Conventional Cellulase and Growth Promoting Agent

468 days old chickens were placed into 18 pens each containing 26 birds (13 male and 13 female). The pens were randomly divided into 3 groups which received the basal feed (Table 8) with the following supplements:

Group A : No supplement
Group B : Celluclast 1.5 L at 1000 g/ton feed
Group C : Virginianycin at 90 ppm

Feed and water were supplied ad lib and body weight gain, feed intake and feed conversion were measured over the 47 day period of the trial. The results were submitted to statistical analysis as previously described.

The results show (Table 9) that addition of a traditional cellulase preparation from Trichoderma reesei (Celluclast 1.5 L) did not significantly influence the feed intake or weight gain of broiler chickens fed a wheat based diet.

## Table 8

### Feed Composition for Example 5

|  | 0-28 days | 29-49 days |
|---|---|---|
| Wheat | 58.0% | 71.0% |
| Soy bean meal | 28.5% | 15.0% |
| Meat & bone meal | 5.0% | 5.0% |
| Corn gluten | 3.0% | 3.0% |
| Fat | 2.0% | 2.0% |
| Trace minerals/vitamins | 3.5% | 4.0% |
| Crude Protein | 23.8% | 19.0% |
| Energy (Kcal/kg) | 2892 | 2992 |

## Table 9

### Feeding Results

|  | A | B | C |
|---|---|---|---|
| Overall weight gain (kg) | 2.09 | 2.11 | 2.18[a] |
| Overall feed intake (kg) | 4.25 | 4.35 | 4.43[a] |
| FCR | 2.05 | 2.02 | 2.03 |

Values with a superscript a) in a single row are significantly different from the control ($p \leqq 0.05$)

**EXAMPLE 6**

Comparison of the effect of a traditional cellulase (SP 346, commercial cellulase from *Trichoderma reesei)* with SP 343 *(Humicola insolens* cellulase/xylanase, DSM 1800). and SP 431 *(Trichoderma longibrachiatum* xylanase, NRRL 11460)

350 chickens were taken from a commercial flock, at 8 days of age and divided into 7 lots of 50 birds.
The birds were raised in individual cages as previously described in Example 1.
The birds were fed on a basal wheat based feed (Table 10) supplemented with enzymes according to the following scheme :

11

SP 346    0.02% and 0.1%
SP 343    0.02% and 0.1%
SP 431    0.01% and 0.025%

### Table 10

| COMPONENT | % IN THE DIET |
|---|---|
| Wheat | 61.40 |
| Soybean meal 46% C.P. | 22.00 |
| Fishmeal 65% C.P. | 5.00 |
| Meat and Bone meal 50% C.P. | 3.30 |
| Calcium carbonate | 0.61 |
| Salt | 0.28 |
| DL-Methionine 98% | 0.17 |
| L-lysine 78% | 0.04 |
| Animal fat | 6.20 |
| Vitamin/Trace Mineral premix* | 0.50 |
| Chromium oxide | 0.50 |

**Analysis**

| | |
|---|---|
| A.M.E. (Kcal/kg) | 3,120 |
| Protein (%) | 22.15 |
| Sulphur amino acids (%) | 0.92 |
| Lysine (%) | 1.20 |
| Fat (%) | 7.85 |
| Crude fiber (%) | 3.00 |
| Calcium (%) | 0.95 |
| Available Phosphorous (%) | 0.40 |

\* Supplies : monensin 100 ppm, bacitracin 50 ppm

Body weight gain, feed intake and feed:gain ratio were measured for each bird from 8 to 22 days of age. A balance experiment was also carried out in which the apparent digestibility of Dry Matter, Fat and Crude Fiber were determined.

The results (Table 11) show that the performance of those chickens receiving the conventional cellulase (SP 346) was not significantly different to that of the control whereas those birds reciving SP 343 or the higher dose of SP 431 had a significantly improved Feed:Gain ratio.

## Table 11

| | Control | 0.02% SP 343 | 0.1% SP 343 | 0.02% SP 346 | 0.1% SP 346 | 0.01% SP 431 | 0.025% SP 431 |
|---|---|---|---|---|---|---|---|
| | | | | TREATMENTS | | | |
| Weight gain (g) | 519.88 | 525.20 | 514.87 | 523.59 | 508.48 | 520.79 | 528.19 |
| (S.D.) | 36.72 | 42.81 | 38.85 | 49.06 | 43.61 | 38.06 | 44.23 |
| Feed intake (g) | 775.38 | 765.86 | 752.59 | 773.15 | 754.10 | 763.94 | 765.58 |
| (S.D.) | 59.60 | 58.99 | 65.54 | 67.31 | 61.72 | 59.26 | 59.56 |
| Feed:Gain ratio | 1.493 | 1.460 | 1.462 | 1.479 | 1.485 | 1.468 | 1.452 |
| (S.D.) | 0.070 | 0.066 | 0.063 | 0.070 | 0.068 | 0.063 | 0.077 |
| | (100) | (97.8) | (97.9) | (99.1) | (99.5) | (98.3) | (97.2) |
| | a | bc | bc | abc | ab | abc | c |

Values for Feed:Gain ratio with a different letter are significantly different at a probability value $p < 0.5$.

The results in Table 12 show that again only the SP 343 and SP 431 enzymes had a positive effect on the apparent digestibility of Dry Matter, Fat and Crude Fiber.

| Apparent Digestibility (%) | | TREATMENTS | | | | | |
|---|---|---|---|---|---|---|---|
| | Control | 0.02% SP 343 | 0.1% SP 343 | 0.02% SP 346 | 0.1% SP 346 | 0.01% SP 431 | 0.025% SP 431 |
| Dry matter | 73.31 | 76.16 | 75.31 | 74.20 | 72.64 | 75.12 | 74.07 |
| (S.D.) | 1.75 | 1.41 | 1.57 | 0.70 | 1.31 | 1.23 | 1.29 |
| | CD | A | AB | BC | D | AB | BC |
| Fat | 82.18 | 84.19 | 85.05 | 83.28 | 81.34 | 84.32 | 85.08 |
| (S.D.) | 59.60 | 58.99 | 65.54 | 67.31 | 61.72 | 59.26 | 59.56 |
| | bc | ab | a | abc | c | ab | a |
| Crude Fiber | 6.13 | 14.22 | 9.28 | 5.56 | 4.04 | 11.49 | 6.48 |
| (S.D.) | 5.64 | 4.82 | 4.52 | 3.10 | 4.56 | 6.10 | 7.87 |
| | C | A | ABC | C | C | AB | BC |

Values for Apparent Dry Matter or Crude Fiber Digestibility with a different letter are significantly different at a probability value $p<0.001$.
Values for Apparent fat Digestibility with a different letter are significantly different at a probability value $p<0.05$.

Claims

1. Feed additive for alleviation of the malabsorption syndrome in poultry, characterized by the fact that it comprises a cellulase and a xylanase producible by means of *Humicola insolens* (DSM 1800) or *Trichoderma longibrachiatum* (NRRL 11460).

2. Poultry feed, characterized by the fact that the poultry feed is composed of a normal poultry feed, to which a feed additive according to Claim 1 has been added.

Patentansprüche

1. Futtermittelzusatz zur Linderung des Malabsorptions-Syndroms bei Geflügel, gekennzeichnet durch die Tatsache, daß er eine Cellulase und eine Xylanase umfaßt, die mittels Humicola insolens (DSM 1800) oder Trichoderma longibrachiatum (NRRL 11460) herstellbar sind.

2. Geflügelfuttermittel, gekennzeichnet durch die Tatsache, daß das Geflügelfuttermittel zusammengesetzt ist aus einem normalen Geflügelfuttermittel, dem ein Futtermittelzusatz gemäß Anspruch 1 zugesetzt worden ist.

**Revendications**

1.  Additif d'alimentation pour atténuer le syndrome de malabsorption de la volaille, caractérisé par le fait qu'il comprend une cellulase et une xylanase pouvant être produites au moyen d'Humicola insolens (DSM 1800) ou de Trichoderma longibrachiatum (NRRL 11460).

2.  Alimentation pour volaille, caractérisée par le fait que l'alimentation pour volaille est constituée par une alimentation normale pour volaille à laquelle on a ajouté un additif d'alimentation selon la revendication 1.